# EUROPEAN PATENT APPLICATION

(11) **EP 4 406 581 A1**
(43) Date of publication of application: **31.07.2024**
(21) Application number: 22916339.9
(22) Date of filing: 24.10.2022
(51) Int. Cl.: A61M 21/02, A61B 5/16, A61B 5/11, A61B 5/00, G16H 20/70, G16H 50/30, G16H 50/20, G16H 10/20

(54) **METHOD FOR DELIVERING DIGITAL THERAPEUTICS**

(30) Priority: 29.12.2021 KR 20210191219
(71) Applicant: IUCF-HYU (Industry-University Cooperation Foundation Hanyang University), Seoul 04763 (KR)
(72) Inventor: KIM, Hyung Sook, Seoul 04763 (KR)
(74) Representative: Prüfer & Partner mbB Patentanwälte · Rechtsanwälte
(86) International application number: PCT/KR2022/016260
(87) International publication number: WO 2023/128185

(57) **Abstract**

Disclosed is a method for delivering digital therapeutics. A method for delivering digital therapeutics according to an embodiment of the present invention may comprise the steps of: determining the degree of emotional disorder of a patient on the basis of diagnosis information and digital therapeutics usage information of the patient; determining digital therapeutics to be delivered to the patient according to the determined degree of emotional disorder, and delivering the determined digital therapeutics to a user terminal of the patient.

## Description

### [Technical Field]

The present invention relates to a method for delivering personalized digital therapeutics (DTX), and more particularly, to a method for delivering digital therapeutics developed as an interactive game and the like in a personalized manner according to a degree of disease of each patient.

### [Background Art]

Recently, a new type of digital treatment technology has been emerging. Digital therapeutics refers to a technology for managing or treating a disease or a disorder by using advanced, evidence-based software. The Ministry of Food and Drug Safety of Korea defines digital therapeutics as "Software as a Medical Device (SaMD)".

Similar to existing compound-based therapeutics, digital therapeutics has a commonality with the compound-based therapeutics in that the digital therapeutics may provide prevention and treatment of diseases and relief of symptoms based on clinically proven evidences. However, since the digital therapeutics uses an ICT technology in a delivery method for the digital therapeutics, enables continuous monitoring of a condition of a patient, and enables non-face-to-face treatment, the digital therapeutics may present a new paradigm especially in treatment of emotional disorders and chronic diseases.

Due to the above characteristics, there is a demand for a technology that may effectively prescribe and deliver digital therapeutics. However, conventionally, technologies have been developed, merely focusing on hardware devices such as a head-mounted display for VR and a haptic glove, which are worn by a user when VR content and the like that constitute the digital therapeutics are used. In addition, a technology for determining a degree of emotional disorder based on new data such as movement data of a patient using digital therapeutics did not exist.

### [Disclosure]

### [Technical Problem]

The present invention is intended to solve the problems described above, and an object of the present invention is to provide a method for delivering digital therapeutics, capable of identifying a degree of a disease of each patient, and transmitting digital therapeutics corresponding to the identified degree of the disease to the patient in order to deliver digital therapeutics suitable for each individual. In addition, an object of the present invention is to provide digital therapeutics in the form of interactive content for treating an emotional disorder, which may be delivered through the method for delivering the digital therapeutics.

### [Technical Solution]

To achieve the objects described above, according to one embodiment of the present invention, there is provided a method for delivering digital therapeutics, the method including: identifying a degree of emotional disorder of a patient based on diagnosis information and digital therapeutics usage information of the patient; determining digital therapeutics to be delivered to the patient according to the identified degree of emotional disorder; and delivering the determined digital therapeutics to a user terminal of the patient.

In addition, the identifying may include: delivering a diagnosis test corresponding to a disease code of the patient; and generating the diagnosis information of the patient by quantifying a result of the diagnosis test.

In addition, the diagnosis test may include items visualized to allow the patient to intuitively understand an emotional state of the patient, and an auditory effect may be assigned for each of the items.

In addition, in the identifying, the degree of emotional disorder of the patient may be identified by using an emotional disorder artificial intelligence model that uses the diagnosis information and the digital therapeutics usage information of the patient as input data.

In addition, the identifying may include: detecting a movement of the patient using the digital therapeutics; extracting feature data associated with the degree of emotional disorder based on the detected movement of the patient; and identifying the degree of emotional disorder of the patient by using the emotional disorder artificial intelligence model based on the feature data.

In addition, the extracting of the feature data may include: analyzing at least one movement data among a reaction time, a movement range, movement speeds of upper and lower extremities, balance, a digital therapeutics execution time, an execution time for each required movement, and a required task success state based on the detected movement of the patient; and extracting the feature data associated with the degree of emotional disorder of the patient based on the analyzed at least one movement data.

In addition, the determining may include: selecting a content set based on the identified degree of emotional disorder; and generating the digital therapeutics to be delivered to the patient by extracting content corresponding to the selected content set.

In addition, in the selecting of the content set, the content set corresponding to the identified degree of emotional disorder may be selected by using a personalized matrix generated based on a type and difficulty of content constituting the digital therapeutics.

In addition, the content set may be configured in an order of base difficulty content of a starting level, enhanced difficulty content of the starting level, activation difficulty content of the starting level, and activation difficulty content of a level higher than the starting level.

In addition, the content constituting the content set may include physical activity content generated based on movement codes corresponding to a plurality of movements to be performed by the patient in order to treat the emotional disorder.

In addition, the physical activity content may be generated by deriving a behavioral inhibition system behavior based on an emotional disorder diagnosis criterion, setting a behavioral activation system behavior pattern objective capable of inducing a change in the derived behavior, and implementing a training movement required to achieve the set behavior pattern objective as interactive content.

In addition, the interactive content may be configured to: display an avatar imitating a detected movement of the patient on a screen; provide a required task that requires touching an object having an instructed shape and an instructed color among a plurality of objects having shapes and colors and approaching a periphery of the avatar; and analyze a reaction time of the patient, a movement range of an upper extremity, and a movement speed of the upper extremity by detecting the movement of the patient performing the required task.

In addition, the interactive content may be configured to: display an avatar imitating a detected movement of the patient on a screen; provide a required task that requires a hand or a foot of the avatar to move while sequentially touching a plurality of points that are away from the avatar; and analyze a movement range and balance of the patient by detecting the movement of the patient performing the required task.

In addition, the interactive content may be configured to: display an avatar imitating a detected movement of the patient on a screen; provide a required task that requires the avatar to follow an instruction to be performed or a movement shown by a guide avatar; and analyze movement speeds of upper and lower extremities of the patient by detecting the movement of the patient performing the required task.

In addition, the interactive content may be configured to: display an avatar imitating a detected movement of the patient on a screen; provide a required task that requires the avatar to move to one of a plurality of sequentially instructed stepping stones while maintaining balance against a preset inclination; and analyze balance of the patient by detecting the movement of the patient performing the required task.

In addition, the interactive content may be configured to: display an avatar imitating a detected movement of the patient on a screen; provide a required task that requires the avatar to run at a preset speed for a preset time; and analyze movement speeds of upper and lower extremities of the patient by detecting the movement of the patient performing the required task.

In addition, the interactive content may be configured to: display an avatar imitating a detected movement of the patient on a screen; provide a required task that requires the avatar to create and maintain a preset movement or move in sequentially instructed directions; and analyze a reaction time of the patient by detecting the movement of the patient performing the required task.

In addition, the delivering may include: receiving digital therapeutics prescription information from a hospital server, and storing the digital therapeutics prescription information; receiving digital therapeutics request information from the user terminal of the patient, and determining whether the digital therapeutics request information corresponds to the stored digital therapeutics prescription information; performing user authentication when the digital therapeutics request information corresponds to the stored digital therapeutics prescription information; and delivering the determined digital therapeutics to the user terminal of the patient when the user authentication is successful.

In addition, the delivering may include: receiving digital treatment environment information from the user terminal of the patient; modifying the determined digital therapeutics based on the received digital treatment environment information; and delivering the modified digital therapeutics to the user terminal of the patient.

### [Advantageous Effects]

According to various embodiments of the present invention as described above, digital therapeutics set in a customized manner based on a diagnosis result and digital therapeutics usage information of each patient can be easily delivered. In addition, a patient can be encouraged to voluntarily perform physical activities that are effective in improving emotional disorders, chronic diseases, and the like by using digital therapeutics in the form of interactive content according to various embodiments of the present invention.

### [Description of Drawings]

FIG. 1 is a block diagram for describing a digital therapeutics delivery system according to one embodiment of the present invention.
FIG. 2 is a block diagram schematically showing a configuration of a digital therapeutics delivery device according to one embodiment of the present invention.
FIG. 3 is a view illustrating a diagnosis test program according to one embodiment of the present invention.
FIG. 4 is a view schematically showing an emotional disorder artificial intelligence model according to one embodiment of the present invention.
FIG. 5 is a view illustrating a personalized matrix for a patient with a depressive disorder according to one embodiment of the present invention.
FIGS. 6 to 11 are views illustrating interactive content that induces a patient with an emotional disorder to perform physical activities according to various embodiments of the present invention.
FIG. 12 is a flowchart for describing a method for delivering digital therapeutics according to one embodiment of the present invention.
FIG. 13 is a sequence diagram for describing the method for delivering the digital therapeutics according to one embodiment of the present invention.

### [Mode for Invention]

Hereinafter, various embodiments of the present disclosure will be described with reference to the accompanying drawings. However, the technology described in the present disclosure is not intended to be limited to a specific embodiment, and is to be understood to encompass various modifications, equivalents, and/or alternatives of an embodiment of the present disclosure. Regarding the description of the drawings, like reference numerals may be used for like elements.

In the present disclosure, an expression such as "has", "may have", "includes", or "may include" refers to the presence of a feature (e.g., an element such as a numerical value, a function, an operation, or a component) corresponding to the expression without precluding the presence of additional features.

In the present disclosure, an expression such as "A or B", "at least one of A and/or B", or "one or more of A and/or B" may include all possible combinations of items enumerated together with the expression. For example, the expression "A or B", "at least one of A and B", or "at least one of A or B" may refer to all cases in which (1) at least one A is included, (2) at least one B is included, or (3) both at least one A and at least one B are included. Expressions such as "first", "second", "firstly", or "secondly" used in the present disclosure may modify various elements regardless of an order and/or importance, and are used only to distinguish one element from another element without limiting the elements corresponding to the expressions.

An expression "configured to (or set to)" used in the present disclosure may be used interchangeably with, for example, "suitable for", "having the capacity to", "designed to", "adapted to", "made to", or "capable of" depending on the circumstances. The term "configured to (or set to)" may not necessarily mean only "specifically designed to" in terms of hardware. Instead, in some circumstances, an expression "a device configured to" may mean that the device is "capable of" something together with other devices or components. For example, "a processor configured to (or set to) perform A, B, and C" may refer to a dedicated processor (e.g., an embedded processor) for performing the operations, or a generic-purpose processor (e.g., a CPU or an application processor) capable of performing the operations by executing one or more software programs stored in a memory device.

Hereinafter, the present invention will be described in detail with reference to the drawings.

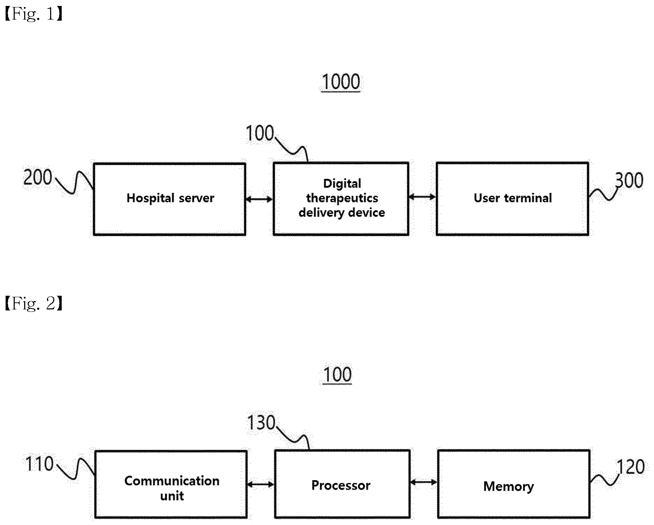

FIG. 1 is a block diagram for describing a digital therapeutics delivery system 1000 according to one embodiment of the present invention. Referring to FIG. 1, the digital therapeutics delivery system 1000 may include a digital therapeutics delivery device 100, a hospital server 200, and a user terminal 300.

The digital therapeutics delivery device 100 may deliver personalized digital therapeutics to a user (patient) to which digital therapeutics is prescribed by a medical institution such as a hospital. The digital therapeutics delivery device 100 may identify a degree of emotional disorder of the patient based on diagnosis information and digital therapeutics usage information of the patient, which are received from the hospital server 200 or the user terminal 300. In addition, the digital therapeutics delivery device 100 may determine digital therapeutics to be delivered to the patient according to the identified degree of emotional disorder. Subsequently, the digital therapeutics delivery device 100 may deliver the determined digital therapeutics to the user terminal 300 of the patient so that the patient may use the digital therapeutics. Specific details of the identification of the degree of emotional disorder of the patient, specific details of the determination of the digital therapeutics to be delivered to the patient, and specific details of the delivery of the digital therapeutics will be described below.

The hospital server 200 may share various data on the patient with the digital therapeutics delivery device 100. As for a data sharing method, the hospital server 200 may transmit data to the digital therapeutics delivery device 100, or the hospital server 200 and the digital therapeutics delivery device 100 may share the same cloud storage space.

The hospital server 200 may transmit at least one of a disease code of the patient, personal information for patient authentication, diagnosis information, digital therapeutics usage information, and digital therapeutics selection information to the digital therapeutics delivery device 100. For example, the hospital server 200 may transmit a disease classification code (disease code) classified by the World Health Organization or the like, patient identification number, resident registration number, and the like to the digital therapeutics delivery device 100. In addition, the hospital server 200 may transmit diagnosis information generated based on a result of diagnosing the user (patient) in a face-to-face or non-face-to-face manner to the digital therapeutics delivery device 100. Furthermore, the hospital server 200 may transmit a hospital EMR and health examination data, such as medical record data, family history data, personal history data, physical fitness data, and body composition data of the patient, which are stored in the hospital server 200, to the digital therapeutics delivery device 100.

In addition, the hospital server 200 may store information on previous digital therapeutics usage of the patient. The digital therapeutics usage information may not be simply usage history information, but may be data obtained by detecting a movement of the patient using the digital therapeutics, or data such as a reaction time, a movement range, movement speeds of upper and lower extremities, balance, a digital therapeutics execution time, an execution time for each required movement, and a required task success state, which are generated based on the movement detection data. In addition, the hospital server 200 may store the digital therapeutics selection information. The digital therapeutics selection information may include information that a specific function such as inducement of physical activities has to be included, information that digital therapeutics including content having visual or auditory stimulation that is greater than or equal to a specific value has to be excluded, and the like.

In other words, the hospital server 200 may interwork with the digital therapeutics delivery device 100, so that basic data for the digital therapeutics delivery device 100 to identify the degree of emotional disorder of the patient to determine suitable digital therapeutics may be provided. Information on the digital therapeutics determined by the digital therapeutics delivery device 100 may be transmitted back to the hospital server 200, so that the digital therapeutics may be prescribed to the patient through final determination of a medical staff.

The user terminal 300 may allow the patient to perform a function of requesting digital therapeutics and a function of executing received digital therapeutics. The user terminal 300 may be configured as general-purpose hardware capable of executing digital therapeutics according to a type of the digital therapeutics and recognizing an execution movement of the patient. For example, the user terminal 300 may be configured as a combination of general-purpose hardware such as a PC, a monitor, Azure Kinect, a camera, a smart phone, and a tablet.

The digital therapeutics delivery device 100 may include elements as in an embodiment of FIG. 2. The digital therapeutics delivery device 100 may include a communication unit 110, a memory 120, and a processor 130. However, in addition to the elements described above, various elements such as an input unit (not shown) and an output unit (not shown) may be additionally included.

The communication unit 110 may communicate with an external device such as the hospital server 200 and the user terminal 300 by using a wired or wireless network. For example, the communication unit 110 may transmit the digital therapeutics to the user terminal 300, and receive information of the patient from the hospital server 200. In addition, the communication unit 110 may receive the digital therapeutics from a digital therapeutics manufacturer. As wireless communication schemes, the communication unit 110 may use various schemes such as Bluetooth, Zigbee communication, Wi-Fi, infrared (IR) communication, and near field communication (NFC), and may be connected to a mobile communication network according to various mobile communication standards such as 3G, 3GPP, LTE, LTE-A, and 5G to perform communication. In addition, as wired communication schemes, the communication unit 110 may use high definition multimedia interface (HDMI), low voltage differential signaling (LVDS), local area network (LAN), and the like.

The memory 120 may store various modules, software, artificial intelligence models, and data for driving the digital therapeutics delivery device 100. For example, the memory 120 may store various types of digital therapeutics, and may store various information such as software for combining digital therapeutics in a personalized manner, software for user authentication, an artificial intelligence model capable of determining a degree of emotional disorder based on movement data of a patient, digital therapeutics usage information, and payment information.

The memory 120 may be implemented in the form of a flash memory, a hard disk drive (HDD), a solid state drive (SSD), or the like. For example, the memory 120 may include a ROM for storing a program and/or an application for performing an operation of the digital therapeutics delivery device 100, and a RAM for temporarily storing data according to the performing of the operation of the digital therapeutics delivery device 100. In addition, the memory 120 may further include an electrically erasable and programmable ROM (EEPROM) for storing various reference data, and the like.

The processor 130 may control the above-described components of the digital therapeutics delivery device 100. For example, the processor 130 may control the communication unit 110 to transmit the personalized digital therapeutics to the user terminal 300. The processor 130 may be manufactured in the form of one or a plurality of hardware chips, and mounted on the digital therapeutics delivery device 100. For example, the processor 130 may be manufactured as a general-purpose processor (e.g., a CPU or an application processor), or may be manufactured in the form of a dedicated hardware chip for artificial intelligence.

The processor 130 may identify the degree of emotional disorder of the patient based on the diagnosis information and the digital therapeutics usage information of the patient. This is to deliver customized digital therapeutics based on the degree of emotional disorder of the patient.

For example, the diagnosis information or the digital therapeutics usage information of the patient may be provided from the hospital server 200. When the medical staff inputs the result of diagnosing the patient online or offline through the EMR of the hospital, the hospital server 200 may transmit the information to the digital therapeutics delivery device 100.

As another example, the digital therapeutics delivery device 100 may generate the diagnosis information of the patient. First, the digital therapeutics delivery device 100 may receive the disease code of the patient from the hospital server 200. The processor 130 may control the communication unit 110 to transmit a diagnosis test corresponding to the disease code of the patient to the user terminal 300. In addition, when a result of the diagnosis test is received from the user terminal 300 through the communication unit 110, the processor 130 may quantify the result of the diagnosis test to generate the diagnosis information of the patient.

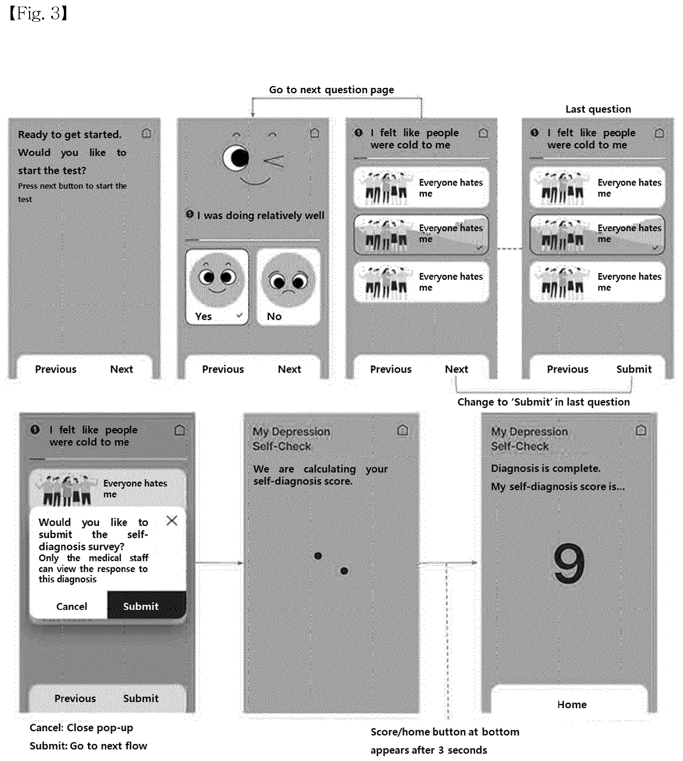

The memory 120 may store a diagnosis test program corresponding to each disease code. For example, each diagnosis test program may be a digitized version of a test sheet designed based on each disease diagnosis criterion presented in Diagnostic & Statistical Manual of Mental Disorders 5^{th} Ed (DSM-5). In particular, in a case of a patient with an emotional disorder, diagnosis may be accurately performed only when the patient may intuitively understand an emotional state of the patient and respond. When taking the above point into consideration, the diagnosis test according to one embodiment of the present invention may visualize and provide items to allow the patient to intuitively understand the emotional state or a physical state of the patient. For example, as in a depressive disorder diagnosis test program shown in FIG. 3, an emotional state asked in each of the items may be configured as a visual element such as an emoticon, an icon, and an infographic so as to be intuitively understood and selected by the patient. In addition, the diagnosis test may be configured such that a question is read for each of the items, or an auditory effect corresponding to the emotional state is assigned.

As in an embodiment of FIG. 3, when the result of the diagnosis test is received through the communication unit 110, the processor 130 may generate the diagnosis information of the patient by deriving a quantified diagnosis score. A quantification scheme may be configured differently according to characteristics of each disease code. For example, in a case of attention deficit hyperactivity disorder (ADHD), the quantified diagnosis score may be derived by assigning a weight for each diagnosis item, and in a case of a depressive disorder, the quantified diagnosis score may be derived by assigning the same weight for each diagnosis item.

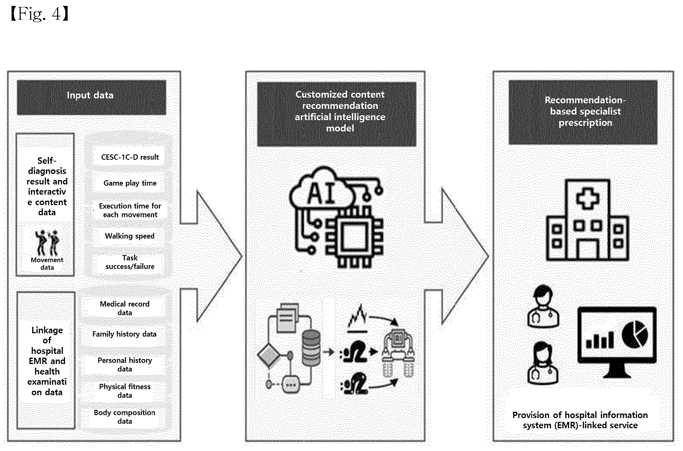

The processor 130 may use an emotional disorder artificial intelligence model to identify the degree of emotional disorder of the patient. The emotional disorder artificial intelligence model may be a model configured to predict the degree of emotional disorder of the patient based on the diagnosis information and the digital therapeutics usage information of the patient as in an embodiment of FIG. 4. In addition, the emotional disorder artificial intelligence model may further use medical record data, family history data, personal history data, physical fitness data, and body composition data of the patient as additional input data. The input data may be processed so that the emotional disorder artificial intelligence model may provide a result of identifying the degree of emotional disorder of the patient as output data. The emotional disorder artificial intelligence model may be a model based on a neural network. For example, a scheme such as a deep neural network (DNN), a recurrent neural network (RNN), or a bidirectional recurrent deep neural network (BRDNN) may be applied, but the present invention is not limited thereto.

The processor 130 may generate the emotional disorder artificial intelligence model by training a criterion for identifying the degree of emotional disorder based on additional information such as the digital therapeutics usage information, the diagnosis information of the patient, and the medical record data. In addition, the processor 130 may identify the degree of emotional disorder of the patient by using the emotional disorder artificial intelligence model that has been trained. The processor 130 may additionally store data, such as the digital therapeutics usage information that is newly input in a process of identifying the degree of emotional disorder by the generated emotional disorder artificial intelligence model, in the memory 120. In addition, the processor 130 may update the generated emotional disorder artificial intelligence model by using additionally stored input data.

As described above, the digital therapeutics usage information may be the data obtained by detecting the movement of the patient using the digital therapeutics or analysis data generated based on the movement detection data. The user terminal 300 may detect and store the movement of the patient when the patient uses the digital therapeutics. The movement detection data may be stored in the form of an RGBD image, or stored in a simplified form such as a skeleton form. The movement detection data may be received through the communication unit 110, and stored in the memory 120. The processor 130 may generate data such as a reaction time, a movement range, movement speeds of upper and lower extremities, balance, a digital therapeutics execution time, an execution time for each required movement, a required task success state, and a walking speed based on the movement detection data.

Since various data may be generated based on the movement detection data of the patient, the processor 130 may determine which data is more associated with the degree of emotional disorder by using the emotional disorder artificial intelligence model. For example, the emotional disorder artificial intelligence model may calculate what movement data affects the identification of the degree of emotional disorder for each emotional disorder disease code during a training stage. The processor 130 may output which data corresponds to feature data that has the greatest influence by using an explainable artificial intelligence (XAI) technology.

The processor 130 may extract feature data associated with the degree of emotional disorder among data generated based on the movement of the patient. In addition, the processor 130 may identify the degree of emotional disorder of the patient by using the emotional disorder artificial intelligence model based on the extracted feature data. The emotional disorder artificial intelligence model may analyze at least one movement data among a reaction time, a movement range, movement speeds of upper and lower extremities, balance, a digital therapeutics execution time, an execution time for each required movement, and a required task success state based on the detected movement of the patient. In addition, the feature data associated with the degree of emotional disorder of the patient may be extracted based on the analyzed at least one movement data.

The processor 130 may determine the digital therapeutics to be delivered to the patient according to the identified degree of emotional disorder. This is because customized digital treatment may be performed only when a usage time, required task difficulty, a required movement type, and the like required for the patient vary depending on the degree of emotional disorder.

According to one embodiment of the present invention, the digital therapeutics delivered to the patient may be physical activity content that induces a plurality of movements to be performed by the patient in order to treat the emotional disorder. In addition, the digital therapeutics may include interactive content that encourages the patient with the emotional disorder to voluntarily perform physical activities. The interactive content may be content that allows the user to actively participate rather than passively receiving information. In other words, the interactive content may refer to content that enables two-way communication based on content that is changed according to an action of the user, rather than content provided in one direction. Since elements of the content react according to the action of the user, the participation of the user may be induced, and immersion and empathy may be increased. The digital therapeutics having such elements may provide a greater improvement effect to the patient with the emotional disorder. For example, an avatar or an object imitating a movement of the user may be displayed on a screen, so that the user may be induced to more actively perform the physical activity content by projecting the user to the avatar displayed on the screen.

In order to generate the physical activity content that induces the movements to be performed by the patient to treat the emotional disorder, movement codes corresponding to the movements may be used. The movement code may be a standardized code capable of expressing quality as well as a form of a movement of a target. For example, the movement code may be generated by classifying and analyzing a movement of a recognized target by characteristics. The movement code may include indicators that objectively express movements that are externally expressed by an intention of the target. For example, the movement code may include indicators that express a weight, a time, a space, and a flow. Therefore, when the movement code is used, it is possible to objectively determine whether the movement is light or strong, whether the movement is sudden or sustained, whether the movement is gazing (direct) or non-gazing (indirect), and whether the movement is free-flowing or bound.

A movement that has to be implemented as the interactive content in the physical activity content may be derived based on an emotional disorder diagnosis criterion. For example, the emotional disorder diagnosis criterion may be a diagnosis criterion for each emotional disorder presented in DSM-5. The processor 130 may derive a behavioral inhibition system behavior based on the emotional disorder diagnosis criterion. In addition, the processor 130 may set a behavioral activation system behavior pattern objective capable of inducing a change in the derived behavioral inhibition system behavior. For example, an indicator on a movement code of the derived behavioral inhibition system behavior may be set as the behavioral activation system behavior pattern objective based on an opposite behavior. Subsequently, the processor 130 may implement a training movement required to achieve the set behavior pattern objective as the interactive content. Alternatively, the processor 130 may provide a guide for generating the interactive content by outputting the set behavior pattern objective, the required training movement, a physical fitness element improved through the training movement, and the like.

As described above, the usage time, the required task difficulty, the required movement type, and the like required for the patient have to vary depending on the identified degree of emotional disorder, so that the processor 130 may select a content set based on the identified degree of emotional disorder. In addition, the processor 130 may generate the digital therapeutics to be delivered to the patient by extracting content corresponding to the selected content set.

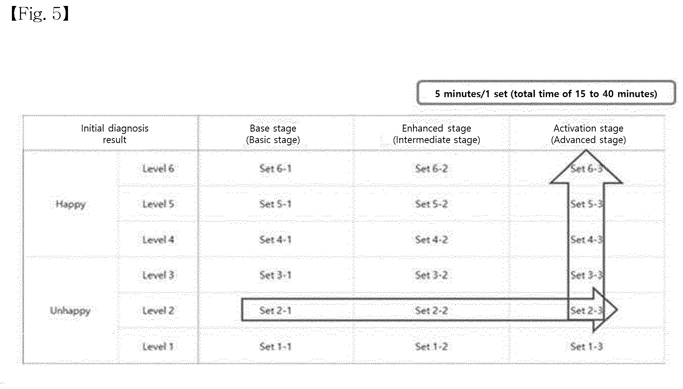

For example, the processor 130 may select the content set by using a personalized matrix generated based on a type or difficulty of content constituting the digital therapeutics. The personalized matrix may be generated based on a total physical activity content provision time required to perform the physical activity content by the patient, a time for each stage (interactive content) constituting the physical activity content, and the number of emotional disorder degree classification levels according to characteristics of each emotional disorder. In addition, one of three factors, which are the total physical activity content provision time, the time for each stage, and the number of levels, may be determined by the remaining factors. When a personalized matrix for a patient with a depressive disorder shown in FIG. 5 is taken as an example, characteristics of the patient with the depressive disorder who do not desire to move may be reflected, so that each stage (interactive content) may be set to 5 minutes, which is a time in which concentration may be maintained. In addition, the total physical activity content provision time may be set to 15 minutes to 40 minutes a day, which is a time in which muscle strength and cardiorespiratory fitness, which are key to improving the depressive disorder, may be effectively improved. In other words, three to eight stages have to be provided according to a depressive disorder degree level, and to this end, the number of depressive disorder degree classification levels may be set to 6.

The content set constituting the physical activity content may include base difficulty content, enhanced difficulty content, and activation difficulty content for each level. This is based on the principle of gradual improvement of physical activities, and the physical activity content may be configured to perform activation difficulty of a different level without the need to be decreased back to the base or enhanced difficulty once the activation difficulty is reached. Referring to a process of selecting a content set to be provided to a patient corresponding to Level 2 among depressive disorder degrees classified into six levels in FIG. 5, the content set may be selected and configured to sequentially provide base difficulty content of Level 2, which is a starting level, enhanced difficulty content of Level 2, activation difficulty content of Level 2, and activation content of Levels 3 to 6, which are levels higher than the starting level.

Hereinafter, six types of interactive content will be illustrated and described. Each interactive content may provide a required task configured to allow the patient to naturally perform a movement that has to be performed to improve the emotional disorder. In addition, the interactive content may be configured to collect movement data that may be used to determine an emotional disorder improvement degree by detecting the movement of the patient performing the required task.

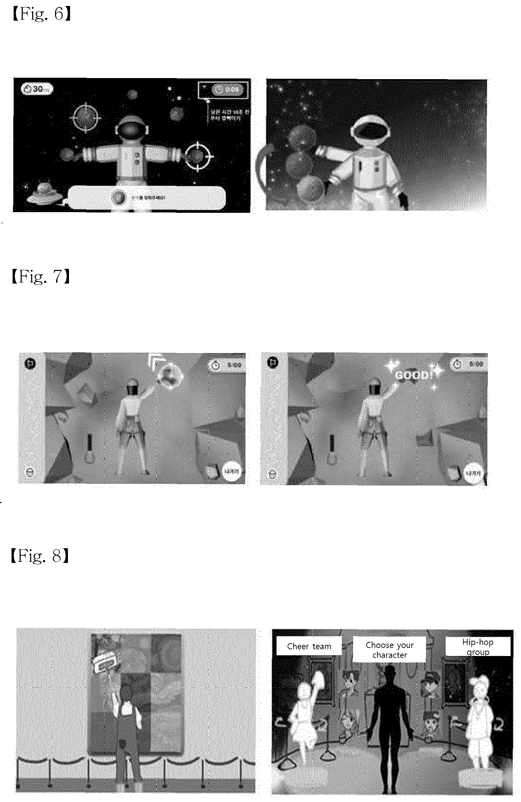

FIG. 6 shows an example of interactive content that induces physical activities so that the patient with the emotional disorder may concentrate on something other than an emotion of the patient. Referring to FIG. 6, an avatar imitating a detected movement of the patient may be displayed on a screen. In addition, objects may approach a periphery of the avatar, and the objects may be set to have a plurality of shapes and colors. The interactive content may provide a required task that requires touching an object having an instructed shape and an instructed color among the objects having the shapes and the colors. A movement of touching the object may be performed according to the provided required task, so that the patient with the emotional disorder may naturally perform the movement that has to be performed to improve the emotional disorder. In particular, in an embodiment of FIG. 6, movement data that may be used to determine the emotional disorder improvement degree such as a reaction time, a movement range of an upper extremity, and a movement speed of the upper extremity may be collected by detecting and analyzing the movement of the patient performing the required task.

FIG. 7 shows an example of interactive content that induces physical activities requiring immersion and concentration of the patient with the emotional disorder. Referring to FIG. 7, an avatar imitating a detected movement of the patient may be displayed on a screen. In addition, a plurality of points that are away from a hand or a foot of the avatar by a preset distance in a preset direction may be displayed. The interactive content may provide a required task that requires the hand or the foot of the avatar to move while sequentially touching the points that are away from the avatar. In particular, in an embodiment of FIG. 7, movement data that may be used to determine the emotional disorder improvement degree such as movement ranges of upper and lower extremities and balance may be collected by detecting and analyzing the movement of the patient performing the required task.

FIG. 8 shows an example of interactive content that induces physical activities that require the patient with the emotional disorder to follow a given instruction. Referring to FIG. 8, an avatar imitating a detected movement of the patient may be displayed on a screen. The interactive content may provide a required task that requires the avatar to follow an instruction to be performed or a movement shown by a guide avatar. The guide avatar may be an avatar that performs a function of showing an instruction through a moving form without being an avatar that imitates the movement of the patient. In particular, in an embodiment of FIG. 8, movement data that may be used to determine movement speeds and movement ranges of upper and lower extremities may be collected by detecting and analyzing the movement of the patient performing the required task.

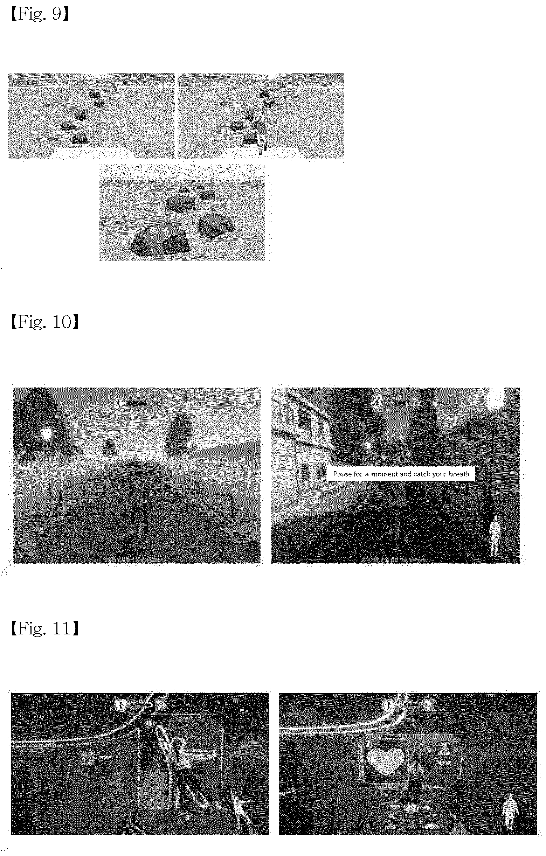

FIG. 9 shows an example of interactive content that induces physical activities that require the patient with the emotional disorder to sequentially move to stepping stones having preset inclinations. Referring to FIG. 9, an avatar imitating a detected movement of the patient may be displayed on a screen. In addition, a plurality of stepping stones that are away from the avatar by a preset distance in a preset direction may be displayed. Each of the stepping stones may have a preset inclination. The interactive content may provide a required task that requires the avatar to move to one of a plurality of sequentially instructed stepping stones while maintaining balance against the preset inclination. In particular, in an embodiment of FIG. 9, movement data that may be used to determine balance of the patient may be collected by detecting and analyzing the movement of the patient performing the required task.

FIG. 10 shows an example of interactive content that induces physical activities that require the patient with the emotional disorder to walk or run at a preset speed. Referring to FIG. 10, an avatar imitating a detected movement of the patient may be displayed on a screen. The avatar may be an avatar that replicates a recognized shape of the patient, or may be an avatar that replicates one of preset objects (e.g., a train, a bicycle, or a car). The interactive content may provide a required task that requires the avatar to run at a preset speed for a preset time. In particular, in an embodiment of FIG. 10, movement data that may be used to determine movement speeds of upper and lower extremities of the patient may be collected by detecting and analyzing the movement of the patient performing the required task.

FIG. 11 shows an example of interactive content that induces physical activities that require the patient with the emotional disorder to move actively and initiatively. Referring to FIG. 11, an avatar imitating a detected movement of the patient may be displayed on a screen. In addition, a screen that guides a preset movement to be performed by the avatar and a screen that indicates a direction in which the avatar is to move may be displayed. The interactive content may provide a required task that requires the avatar to create and maintain a preset movement or move in sequentially instructed directions. In particular, in an embodiment of FIG. 11, movement data that may be used to determine a reaction time of the patient may be collected by detecting and analyzing the movement of the patient performing the required task.

The processor 130 may provide the determined digital therapeutics to the user terminal 300 of the patient. A factor that has to be considered when the digital therapeutics is delivered may be user authentication and digital treatment environment information of the patient. This is because the digital therapeutics has to be used only by a patient to which the digital therapeutics is prescribed since because the customized digital therapeutics is delivered, and a delivery method has to vary according to the digital treatment environment information of the patient to ensure stable provision and execution of the digital therapeutics.

The processor 130 may provide information on the determined digital therapeutics to the hospital server 200 to recommend appropriate digital therapeutics to the medical staff. When the medical staff prescribes the recommended digital therapeutics or digital therapeutics changed to reflect clinical determination of the medical staff, the hospital server 200 may transmit digital therapeutics prescription information to the digital therapeutics delivery device 100. When the digital therapeutics prescription information is received through the communication unit 110, the processor 130 may store the received digital therapeutics prescription information in the memory 120.

The digital therapeutics prescription information may include at least one of a disease code, information for prescription patient authentication, digital therapeutics selection information, diagnosis information, and usage history information. The disease code may be also referred to as a disease classification code, which may be based on International Classification of Diseases of the World Health Organization (WHO). The information for the prescription patient authentication may be user information required to give authority to receive the digital therapeutics only to a person to which the digital therapeutics is prescribed. For example, the user information may include various information that may authenticate the user, such as a patient identification number or a resident registration number. The digital therapeutics selection information may include information on the prescribed digital therapeutics. The diagnosis information may be information obtained by diagnosing a patient to which the digital therapeutics is prescribed by a doctor, which may include precautions upon use of the digital therapeutics and the like. The usage history information may include information on what type of digital therapeutics has previously been prescribed to the user (patient). The usage history information may include a follow-up result for use of specific digital therapeutics so as to determine whether the digital therapeutics is effective for the user.

The user terminal 300 may transmit digital therapeutics request information to the digital therapeutics delivery device 100. The hospital server 200 may transmit a message including various schemes such as a URL, a QR code, a bar code, a download link, and a cloud access code to the user terminal 300 to which the digital therapeutics is prescribed. The user terminal 300 may transmit the digital therapeutics request information for the prescribed digital therapeutics to the digital therapeutics delivery device 100 through the various schemes included in the message.

The processor 130 may determine whether the digital therapeutics request information received from the user terminal 300 corresponds to the digital therapeutics prescription information transmitted from the hospital server 200. When the two information correspond to each other, the processor 130 may perform the user authentication so that only the user to which digital therapeutics is prescribed may receive the digital therapeutics. When the user authentication is successful, the processor 130 may control the communication unit 110 to deliver the determined digital c to the user terminal 300 of the patient.

For example, the processor 130 may transmit a digital therapeutics executable file in order to deliver the digital therapeutics to the user terminal 300.

As another example, the processor 130 may control the communication unit 110 to communicate with the user terminal 300, allow execution of the digital therapeutics to be performed through the digital therapeutics delivery device 100 or an external cloud server (not shown), and transmit only files required for visualization on a monitor or the like to the user terminal 300. Use of the above scheme may reduce burden of transmitting the digital therapeutics executable file, which is large, and ensure safety from a risk of hacking. In addition, the execution of the digital therapeutics may be performed on the digital therapeutics delivery device 100 or the external cloud server (not shown), which has excellent computing power, so that burden of requiring the patient to have a high-specification digital treatment environment may be reduced.

As still another example, the processor 130 may distribute tasks so that the user terminal 300 may process the execution of the digital therapeutics in parallel with the digital therapeutics delivery device 100 or the external cloud server (not shown). Which task is to be distributed to which device may be associated with digital treatment environment information, which will be described below.

The processor 130 may transmit a control signal for instructing transmission of the digital treatment environment information to the user terminal 300 of the patient through the communication unit 110. When the digital treatment environment information is received from the user terminal 300 in response to the control signal, the processor 130 may modify the digital therapeutics determined to be delivered based on the received digital treatment environment information. The digital treatment environment information may include specification information such as hardware configuration information of the user terminal 300, computing power of each hardware, resolution, and storage space capacity, operating system (OS) information, and the like.

For example, in a case of downloading the digital therapeutics executable file, the processor 130 may modify the digital therapeutics through a change in resolution of the interactive content, a change in a graphic computing amount, such as removal of a background object, and the like based on the received digital treatment environment information.

As another example, when the execution of the digital therapeutics is performed in the digital therapeutics delivery device 100 or the external cloud server (not shown) (processed alone or in parallel with the user terminal 300), the processor 130 may determine which function to be executed on which device in consideration of computing power of the user terminal 300 and the like. For a task that requires high computing power, such as the generation of the movement data through the analysis of the detected movement of the user or a task that handles data that may be leaked from the user terminal 300, the processor 130 may determine to execute the task on the digital therapeutics delivery device 100. In addition, a task that may be smoothly executed in consideration of the computing power of the user terminal 300 and the like among the remaining tasks may be determined to be processed in the user terminal 300. The processor 130 may generate a digital therapeutics executable file in which only tasks to be processed in the user terminal 300 may be executed, and may control the communication unit 110 to transmit the generated executable file to the user terminal 300.

Hereinafter, a method for delivering digital therapeutics according to various embodiments of the present invention will be described again through a flowchart and a sequence diagram.

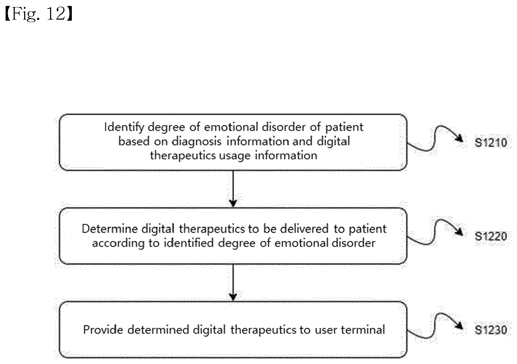

FIG. 12 is a flowchart for describing a method for delivering digital therapeutics according to one embodiment of the present invention. Referring to FIG. 12, a digital therapeutics delivery device 100 may identify a degree of emotional disorder of a patient based on diagnosis information and digital therapeutics usage information of the patient (S1210). In addition, the digital therapeutics delivery device 100 may additionally use medical record data, family history data, personal history data, physical fitness data, and body composition data of the patient to identify the degree of emotional disorder of the patient.

The diagnosis information of the patient may be transmitted from a hospital server 200 to the digital therapeutics delivery device 100, or the digital therapeutics delivery device 100 may autonomously generate the diagnosis information. For example, the digital therapeutics delivery device 100 may deliver a diagnosis test corresponding to a disease code of the patient among a plurality of stored diagnosis tests to a user terminal 300. The delivered diagnosis test may be a digitized version of a test sheet designed based on a disease diagnosis criterion. A diagnosis test program may be configured such that an item asked to allow the patient to intuitively understand an emotional state or a physical state of the patient is visualized as an emoticon, an icon, an infographic, or the like, and an auditory effect is assigned to the item together with the visualization.

When a result of the diagnosis test is received from the user terminal 300, the digital therapeutics delivery device 100 may generate the diagnosis information of the patient by quantifying the result of the diagnosis test. The quantification of the diagnosis test may be a preprocessing process for use as input data in an artificial intelligence model for identifying the degree of emotional disorder. For example, the digital therapeutics delivery device 100 may quantify the test result by assigning a preset weight for each item according to a type of the emotional disorder.

The digital therapeutics delivery device 100 may identify the degree of emotional disorder of the patient by using an emotional disorder artificial intelligence model that uses the diagnosis information of the patient generated or received as described above and the digital therapeutics usage information as input data. The input data may additionally include medical record data, family history data, personal history data, physical fitness data, and body composition data of the patient. In particular, in a case of the digital therapeutics usage information, the digital therapeutics delivery device 100 may extract feature data associated with the degree of emotional disorder based on a movement of the patient detected by the user terminal 300. In addition, the degree of emotional disorder of the patient may be predicted by using the emotional disorder artificial intelligence model based on the extracted feature data. In order to extract the feature data, the digital therapeutics delivery device 100 may generate and analyze movement data such as a reaction time, a movement range, movement speeds of upper and lower extremities, balance, a digital therapeutics execution time, an execution time for each required movement, and a required task success state based on the detected movement of the patient. In addition, the digital therapeutics delivery device 100 may extract the feature data associated with the degree of emotional disorder of the patient based on the movement data. The digital therapeutics delivery device 100 may use an explainable artificial intelligence technique when determining association.

Subsequently, the digital therapeutics delivery device 100 may determine digital therapeutics to be delivered to the patient according to the identified degree of emotional disorder (S1220). The digital therapeutics delivery device 100 may select a content set corresponding to the identified degree of emotional disorder of the patient based on a type and difficulty of content constituting the digital therapeutics. In addition, the digital therapeutics delivery device 100 may generate the digital therapeutics to be delivered to the patient by extracting content corresponding to the selected content set. Each content may be generated based on movement codes corresponding to a plurality of movements to be performed by the patient in order to treat the emotional disorder. When the movement to be performed by the patient (behavior pattern objective) is set, a behavioral inhibition system behavior may be derived based on an emotional disorder diagnosis criterion, and a movement corresponding to a movement code of an index that is opposite to a movement code of the derived behavioral inhibition system behavior may be set to be performed.

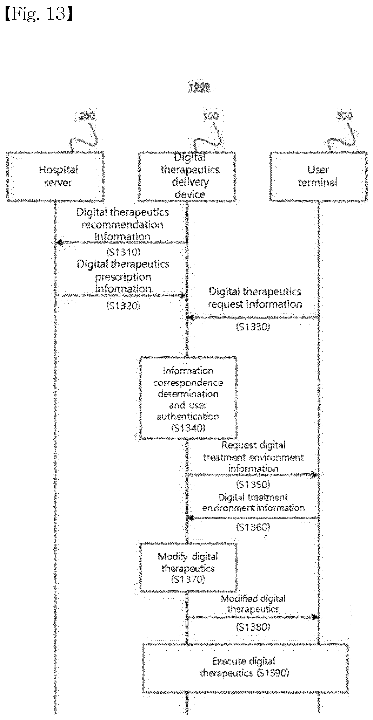

In addition, the digital therapeutics delivery device 100 may deliver the determined digital therapeutics to the user terminal 300 of the patient (S1230). Hereinafter, a method for delivering digital therapeutics will be described with reference to a sequence diagram of FIG. 13. However, the method for delivering the digital therapeutics to a user terminal 300 may include various methods as described above without being limited to an embodiment of FIG. 13. Referring to the embodiment shown in the sequence diagram of FIG. 13, a digital therapeutics delivery device 100 may configure information on determined digital therapeutics as digital therapeutics recommendation information, and transmit the digital therapeutics recommendation information to a hospital server 200 (S1310). The hospital server 200 may transmit digital therapeutics prescription information in which recommended digital therapeutics or digital therapeutics changed based on determination of a medical staff is prescribed to the digital therapeutics delivery device 100 (S1320). In addition, the hospital server 200 may transmit a message including various schemes for requesting the digital therapeutics to a user terminal 300. When digital therapeutics request information is received from the user terminal 300 (S1330), the digital therapeutics delivery device 100 may check whether the digital therapeutics prescription information corresponds to the digital therapeutics request information, and perform user authentication (S1340).

Subsequently, the digital therapeutics delivery device 100 may transmit a control signal for requesting digital treatment environment information to the user terminal 300 (S1350). When the digital treatment environment information is received from the user terminal 300 (S1360), the digital therapeutics delivery device 100 may modify the determined digital therapeutics based on the received digital treatment environment information (S1370). As described above, the modification of the digital therapeutics may mean program optimization, task distribution, or the like to ensure smooth execution of the digital therapeutics. The digital therapeutics delivery device 100 may transmit the modified digital therapeutics to the user terminal 300 (S1380). In addition, the digital therapeutics may be executed on the user terminal 300 alone, or executed in parallel with the digital therapeutics delivery device 100 (S1390).

According to various embodiments of the present invention as described above, digital therapeutics set in a customized manner based on a diagnosis result and digital therapeutics usage information of each patient may be easily delivered. In addition, a patient may be encouraged to voluntarily perform physical activities that are effective in improving emotional disorders, chronic diseases, and the like by using digital therapeutics in the form of interactive content according to various embodiments of the present invention. In addition, the digital therapeutics may be executed smoothly regardless of a level of a digital treatment environment the patient has.

Meanwhile, a term "part" or "module" used in the present disclosure includes a unit configured as hardware, software, or firmware, and may be used interchangeably with, for example, a term such as "logic", "logical block", "component", or "circuit". The term "part" or "module" may refer to an integrated component, a minimum unit that performs one or more functions, or a portion thereof. For example, a module may be configured as an application-specific integrated circuit (ASIC) .

Various embodiments of the present invention may be implemented as software including instructions stored in machine (e.g., a computer)-readable storage media. The machine may be a device capable of calling the stored instructions from the storage medium and operating according to the called instructions, and may include an electronic device (e.g., the digital therapeutics delivery device 100) according to the disclosed embodiments. When the instruction is executed by a processor, the processor may directly perform a function corresponding to the instruction, or perform the function by using other elements under the control of the processor. The instruction may include a code generated or executed by a compiler or an interpreter. The machine-readable storage medium may be provided in the form of a non-transitory storage medium. In this case, the term "non-transitory" only means that the storage medium is tangible without including a signal, and does not distinguish whether data is stored semi-permanently or temporarily in the storage medium.

According to one embodiment, a method according to various embodiments disclosed in the present disclosure may be included and provided in a computer program product. The computer program product may be traded between a seller and a buyer as a commodity. The computer program product may be distributed in the form of a machine-readable storage medium (e.g., compact disc read-only memory (CD-ROM)), or distributed online through an application store (e.g., Play Store^{™}). In a case of the online distribution, at least a portion of the computer program product may be at least temporarily stored or temporarily generated in a storage medium such as a memory of a server of a manufacturer, a server of an application store, or a relay server.

Each of elements (e.g., modules or programs) according to various embodiments may be configured as a single entity or a plurality of entities, in which some sub-elements among previously-described sub-elements may be omitted, or other sub-elements may be further included in various embodiments. Alternatively or additionally, some elements (e.g., modules or programs) may be integrated into a single entity to perform the same or similar function performed by each of the elements before the integration. Operations performed by modules, programs, or other elements according to various embodiments may be executed sequentially, in parallel, iteratively, or heuristically, at least some operations may be executed in a different order or omitted, or other operations may be added.

### [Industrial Applicability]

The technical idea according to an embodiment of the present disclosure may be used as a method for delivering digital therapeutics.

## Claims

1. A method for delivering digital therapeutics, the method comprising:
identifying a degree of emotional disorder of a patient based on diagnosis information and digital therapeutics usage information of the patient;
determining digital therapeutics to be delivered to the patient according to the identified degree of emotional disorder; and
delivering the determined digital therapeutics to a user terminal of the patient.

2. The method of claim 1, wherein the identifying includes:
delivering a diagnosis test corresponding to a disease code of the patient; and
generating the diagnosis information of the patient by quantifying a result of the diagnosis test.

3. The method of claim 2, wherein the diagnosis test includes items visualized to allow the patient to intuitively understand an emotional state of the patient, and an auditory effect is assigned for each of the items.

4. The method of claim 1, wherein, in the identifying, the degree of emotional disorder of the patient is identified by using an emotional disorder artificial intelligence model that uses the diagnosis information and the digital therapeutics usage information of the patient as input data.

5. The method of claim 4, wherein the identifying includes:
detecting a movement of the patient using the digital therapeutics;
extracting feature data associated with the degree of emotional disorder based on the detected movement of the patient; and
identifying the degree of emotional disorder of the patient by using the emotional disorder artificial intelligence model based on the feature data.

6. The method of claim 5, wherein the extracting of the feature data includes:
analyzing at least one movement data among a reaction time, a movement range, movement speeds of upper and lower extremities, balance, a digital therapeutics execution time, an execution time for each required movement, and a required task success state based on the detected movement of the patient; and
extracting the feature data associated with the degree of emotional disorder of the patient based on the analyzed at least one movement data.

7. The method of claim 1, wherein the determining includes:
selecting a content set based on the identified degree of emotional disorder; and
generating the digital therapeutics to be delivered to the patient by extracting content corresponding to the selected content set.

8. The method of claim 7, wherein, in the selecting of the content set, the content set corresponding to the identified degree of emotional disorder is selected by using a personalized matrix generated based on a type and difficulty of content constituting the digital therapeutics.

9. The method of claim 7, wherein the content set is configured in an order of base difficulty content of a starting level, enhanced difficulty content of the starting level, activation difficulty content of the starting level, and activation difficulty content of a level higher than the starting level.

10. The method of claim 7, wherein the content constituting the content set includes physical activity content generated based on movement codes corresponding to a plurality of movements to be performed by the patient in order to treat the emotional disorder.

11. The method of claim 10, wherein the physical activity content is generated by deriving a behavioral inhibition system behavior based on an emotional disorder diagnosis criterion, setting a behavioral activation system behavior pattern objective capable of inducing a change in the derived behavior, and implementing a training movement required to achieve the set behavior pattern objective as interactive content.

12. The method of claim 11, wherein the interactive content is configured to:
display an avatar imitating a detected movement of the patient on a screen;
provide a required task that requires touching an object having an instructed shape and an instructed color among a plurality of objects having shapes and colors and approaching a periphery of the avatar; and
analyze a reaction time of the patient, a movement range of an upper extremity, and a movement speed of the upper extremity by detecting the movement of the patient performing the required task.

13. The method of claim 11, wherein the interactive content is configured to:
display an avatar imitating a detected movement of the patient on a screen;
provide a required task that requires a hand or a foot of the avatar to move while sequentially touching a plurality of points that are away from the avatar; and
analyze a movement range and balance of the patient by detecting the movement of the patient performing the required task.

14. The method of claim 11, wherein the interactive content is configured to:
display an avatar imitating a detected movement of the patient on a screen;
provide a required task that requires the avatar to follow an instruction to be performed or a movement shown by a guide avatar; and
analyze movement speeds of upper and lower extremities of the patient by detecting the movement of the patient performing the required task.

15. The method of claim 11, wherein the interactive content is configured to:
display an avatar imitating a detected movement of the patient on a screen;
provide a required task that requires the avatar to move to one of a plurality of sequentially instructed stepping stones while maintaining balance against a preset inclination; and
analyze balance of the patient by detecting the movement of the patient performing the required task.

16. The method of claim 11, wherein the interactive content is configured to:
display an avatar imitating a detected movement of the patient on a screen;
provide a required task that requires the avatar to run at a preset speed for a preset time; and
analyze movement speeds of upper and lower extremities of the patient by detecting the movement of the patient performing the required task.

17. The method of claim 11, wherein the interactive content is configured to:
display an avatar imitating a detected movement of the patient on a screen;
provide a required task that requires the avatar to create and maintain a preset movement or move in sequentially instructed directions; and
analyze a reaction time of the patient by detecting the movement of the patient performing the required task.

18. The method of claim 1, wherein the delivering includes:
receiving digital therapeutics prescription information from a hospital server, and storing the digital therapeutics prescription information;
receiving digital therapeutics request information from the user terminal of the patient, and determining whether the digital therapeutics request information corresponds to the stored digital therapeutics prescription information;
performing user authentication when the digital therapeutics request information corresponds to the stored digital therapeutics prescription information; and
delivering the determined digital therapeutics to the user terminal of the patient when the user authentication is successful.

19. The method of claim 1, wherein the delivering includes:
receiving digital treatment environment information from the user terminal of the patient;
modifying the determined digital therapeutics based on the received digital treatment environment information; and
delivering the modified digital therapeutics to the user terminal of the patient.
